# EUROPEAN PATENT SPECIFICATION

(11) **EP 3 165 254 B1**
(45) Date of publication and mention of the grant of the patent: **22.08.2018**
(21) Application number: 16194760.1
(22) Date of filing: 20.10.2016
(51) Int. Cl.: A61N 1/05, A61N 1/375

(54) **ANCHORING CONTROL SYSTEM FOR MEDICAL DEVICES**
VERANKERUNGSSTEUERUNGSSYSTEM FÜR MEDIZINISCHE VORRICHTUNGEN
SYSTÈME DE CONTRÔLE D'ANCRAGE POUR DISPOSITIFS MÉDICAUX

(30) Priority: 03.11.2015 US 201562249958 P
(43) Date of publication of application: 10.05.2017
(73) Proprietor: BIOTRONIK SE & Co. KG, 12359 Berlin (DE)
(72) Inventor: Becker, Andreas, Wilsonville, OR Oregon 97070 (US); Muessig, Dirk, West Linn, OR Oregon 97068 (US)
(74) Representative: Galander, Marcus

(56) References cited:
- WO-A1-2005/037368
- WO-A2-01/26706
- US-A- 6 086 582
- US-A1- 2004 122 422

## Description

Embodiments disclosed herein relate to apparatuses and methods for anchoring an implantable leadless pacemaker to body tissue and, in particular, to apparatuses and methods for ascertaining whether the implantable leadless pacemaker is properly seated within and/or attached to body tissue.

Implantable medical devices can be used to monitor, stimulate and/or regulate organs and physiological functions of a living being (e.g, animal, human, etc.). Generally, the medical device is secured by connecting it to body tissue, with the body tissue being the tissue of the body part the medical device is monitoring (sensing), stimulating (pacing) and/or regulating. For example, a pacemaker may be attached to a portion of a heart to obtain signals from, and/or generate signals for functions of, the heart. As another example, a venous access port may be attached to a vein to provide medications to the body via the vein. Attaching an implantable medical device to a body tissue may be referred to as anchoring the device. As a further example, an implantable neurostimulator may be placed in close proximity to a nerve fibre, nerve bundle, spinal cord or other tissue comprising nerve cells to obtain signals from, and/or generate signals to influence the transmitting of signals. Further examples relate to implantable electrode leads like, for example, cardiac stimulation leads, which are associated with, for example, pacemakers.

One of the major problems during implantation of an implantable medical device concerns anchoring the device to body tissue in order to ensure patient safety and good sensing/pacing. Problems exist if the implantable medical device is not anchored well (*i.e,* loosely anchored) to the body tissue. During implantation, perforations of tissue, especially heart tissue, can occur. Clinical trials have shown that one of the most occurred problems concerns the perforation of tissue, for example, cardiac tissue, by over twisting the medical device or applying high pressure during implantation at the implantation site. If a perforation happens during surgery, immediate emergency care must be provided. Other problems relate to insufficient anchoring (*i.e*., not properly seated within and/or attached to the tissue), which can lead to problems during sensing and/or stimulation. In one instance, such situations can occur if the device is not implanted orthogonally.

Depending on the particular implanted device, proper anchoring may take a number of forms. For example, some medical devices may perform optimally when abutting the tissue, but malfunction when not abutting the tissue. Other medical devices may perform optimally when seated within the tissue at a certain depth, but malfunction when not seated within the tissue at a certain depth. Still, other medical devices may perform optimally when attached at a particular distance from the tissue, but malfunction when not attached at a distance from the tissue. These situations can develop due to improper anchoring of the medical device during implantation or shifting of the medical device after implantation.

Further problems can arise if the tissue to which the medical device is attached becomes damaged. Damage to tissue may occur by applying too much pressure while attaching the device to the tissue, resulting in a tear in and/or perforation of the tissue. Damage may also occur after the device is attached if the device is caused to shift, resulting in a tear and/or perforation of the tissue.

Document WO 2005/037368 A1 discloses a medical lead fixation. A distal tip coupled to a body of an implantable medical device includes a canted passageway extending distally from a lumen of the body and an opening terminating the passageway and positioned in proximity to a distal end of the distal tip. A helical fixation element coupled to an elongated member extending within the lumen of the body is adapted to deflect along the canted passageway of the distal tip. The elongated member is adapted to move the helical member through the passageway of the distal tip and out from the opening and to rotate the helical element thereby affixing the helical element into an implant site.

Document US 2004/0122422 A1 discloses an apparatus comprising a helical support adapted to corkscrew into a tissue, and a medical device assembled with the helical support. The medical device is adapted to release a substance, or to emit non-RF energy. The medical device may comprise a non-helical radio frequency (RF) electrode mounted on a distal tip of the helical support.

Document US 6,086,582 A discloses a cardiac drug delivery system for administering a therapeutic agent locally and to a depth within cardiac tissue. An elongate, flexible catheter contains a flexible electric conductor and supports at its distal end an implantable electrode incorporating a penetrating element, typically a fixation helix or a linear needle that penetrates cardiac tissue as the electrode is implanted. A therapeutic agent is delivered through the electrode, to the cardiac tissue surrounding the penetrating element. The electrode acts as a sensor, electrically coupled through the flexible conductor, and monitors an electrical condition of the surrounding cardiac tissue. A controller is coupled to the sensor and to a pump or reservoir containing the therapeutic agent, to control delivery of the agent responsive to the sensed electrical condition. The implanted electrode further can be used to deliver RF current to ablate the surrounding tissue. Several embodiments feature a distal reservoir adjacent the electrode, for effecting transient deliveries of the therapeutic agent in minute quantities or chronic delivery of growth factors. Another embodiment incorporates a bilumen catheter and a set of unidirectional valves, to facilitate changing therapeutic agents or purging the catheter of an agent after delivery.

Document WO 01/26706 A2 discloses a drug delivery catheter suited for cardiac procedures. The catheter includes a distal helical coil or other fixation and penetrating element which can be operated from the proximal end of the catheter to engage and penetrate the myocardium. Once delivered to the inside of the heart, the catheter can be used to inject small doses of therapeutic agents to the myocardium. The drug delivery system of the catheter allows for precise control of the dose injected into the heart wall. The present invention is directed toward overcoming one or more of the above-mentioned problems.

An pacemaker system according to claim 1 is provided. Further embodiments are subject matter of dependent claims.

Embodiments of the disclosure relate to anchors of injectable devices, for example, implantable leadless pacemakers. Additionally, the disclosure relates to a method of controlling the optimal implantation of an implantable device without the perforation of body tissue (*e.g*., heart tissue). The injectable device includes an anchor which is contrast agent enhanced, such that contrast agent may be released from the anchor, either on-demand or self-released after a defined period of time.

The inventive apparatus, as described herein, includes an anchor that may be attachable to an implantable medical device and can be further structured to attach the implantable medical device to body tissue. A proximal portion of the anchor can be configured to temporarily or permanently attach to the implantable medical device, and a distal portion of the anchor can be configured to temporarily or permanently attach to body tissue. The apparatus may further include a reservoir having a contrast agent fluid contained therein. The apparatus may further include a discharge channel having a pathway formed therein to facilitate a flow of fluid (*e.g*., a contrast agent), where the pathway may have an inlet end in fluid connection with a reservoir and an outlet end configured to dispense fluid taken from the reservoir. In a specific embodiment, the discharge channel comprises the reservoir. The pathway of the discharge channel may be routed from the reservoir to the distal portion of the anchor so that the outlet end of the discharge channel can be positioned at or near the distal portion of the anchor. The discharge channel may be formed within the anchor, or may be a separate member attached to the anchor.

During and/or after attaching the implantable medical device to body tissue via the anchor, fluid can be taken from the reservoir and forced to flow through the discharge channel and be dispensed to a volume of space outside of the outlet end. The anchor can be structured such that if the implantable medical device is properly seated within the body tissue and/or attached to the body tissue, the outlet end can be positioned within the body tissue, and fluid can be dispensed into, or at least substantially into, the body tissue. However, if the implantable medical device is not properly seated within the body tissue and/or attached to the body tissue, the outlet end can be positioned outside of the body tissue, and fluid thus dispensed outside the body tissue. Such a situation can occur if, for example, the implantable medical device is seated too far into the body tissue and the distal portion of the anchor perforates the body tissue. In this instance, fluid from the discharge channel would flow outside of the body tissue. As another example, if during implantation, manipulation of the anchor tears the body tissue so as to degrade the structural integrity of the tissue surface at the attachment point, fluid may not be contained by the body tissue, and thus the fluid may be disbursed substantially outside of the body tissue.

During and/or after attaching the implantable medical device to the body tissue via the anchor, imaging of the implantation site via X-ray, Magnetic Resonance Imaging ("MRI"), ultrasound, or other imaging techniques may be performed. The fluid can then be dispensed as the imaging is conducted or before the imaging is conducted. The fluid being dispensed can be a contrast fluid that appears very distinctly in the image due to reactions with the energies emitted by the imaging apparatus (*e.g*., excitations with x-rays, resonant vibrations with magnetic signals, echogenicity with sound waves, etc.). The observance or lack of observance of a distinct appearance that may be caused by the contrast fluid can be used as a proxy for whether the implantable medical device is properly seated within and/or attached to the body tissue. The fluid may be a liquid, a solvable powder or a solvable solid object, or the like.

For instance, if the implantable medical device is properly seated/attached, the contrast fluid may not be able to be dispensed at all, or dispense very slowly, because the tissue is obstructing the flow of fluid from the outlet end, and/or the body tissue may obscure the contrast fluid because it is being substantially contained within the body tissue. Thus, observing no or very little contrast effect can indicate that the implantable medical device may be properly seated/attached. If the implantable medical device is not properly seated/attached, the contrast fluid may dispense quickly and be dispensed outside, or substantially outside, of the tissue-body and into close by cavities. Thus, observing a contrast effect that is more like a scattering and/or diffusing pattern caused by disbursement of the fluid and/or an observance of an image contrast appearing for an unexpected body tissue caused by saturation of the fluid into a neighboring body tissue can indicate that the implantable medical device may not be properly seated/attached.

Use of the apparatus and the disclosed method can not only determine if the implantable medical apparatus is properly seated/attached, but it can also determine if damage to the tissue-body has occurred. Damage to the body-tissue can require urgent care. Thus, the apparatus and method of use can enable medical professionals to ascertain, in real time, whether damage is occurring (*e.g*., a perforation) and/or has occurred so that immediate emergency care can be taken. Damage, such as tissue perforation, may occur due to over-twisting the anchor or applying too much pressure to the anchor when piercing the tissue-body to effectuate the attachment thereto. Damage and/or improper seating/attachment can also be caused by the implantable medical device not being implanted orthogonally.

The inventive apparatus and method can provide an easy-to-perform control mechanism for recognizing fixation problems during and/or after attaching an implantable medical device to tissue via the anchor. Furthermore, optimal implantation can be ensured without harm to the patient.

While these potential advantages are made possible by technical solutions offered herein, they are not required to be achieved. The presently disclosed apparatus and method can be implemented to achieve technical advantages, whether or not these potential advantages, individually or in combination, are sought or achieved.

It is an object of the present disclosure to provide an easy-to-perform control mechanism for recognizing fixation problems in implantable medical devices.

It is a further object of the present disclosure to ensure optimal implantation of an implantable medical device without harm to the patient.

In one embodiment of the inventive apparatus, an anchoring control system comprises at least one anchor having a proximal portion affixed to an implantable medical device and a distal portion capable of being affixed to body tissue, a reservoir having a contrast agent fluid contained therein, and at least one discharge channel associated with the at least one anchor, the at least one discharge channel being in fluid communication with the reservoir and having an outlet at the distal portion of the at least one anchor, wherein contrast agent fluid is provided through the discharge channel and output at the outlet to determine whether the implantable medical device is properly affixed to the body tissue.

In this embodiment, the reservoir may be disposed within the implantable medical device. Additionally, or alternatively, the reservoir may be attached to the implantable medical device, either permanently or temporarily.

In one variant of the above mentioned anchoring control system, the reservoir may comprise a compressible, fluid absorbent member attached to the implantable medical device, wherein compression of the member forces contrast agent fluid through the discharge channel and outlet. The compressible, fluid absorbent member of this variant may comprise, for example, a sponge.

In an alternative embodiment of the inventive apparatus, an anchoring control system comprises at least one anchor having a proximal portion affixed to an implantable medical device and a distal portion capable of being affixed to body tissue, and at least one discharge channel associated with the at least one anchor, the at least one discharge channel having an outlet at the distal portion of the at least one anchor, a pathway and a reservoir, wherein the pathway is in fluid communication with the reservoir and the outlet, wherein contrast agent fluid is provided through the discharge channel and output at the outlet to determine whether the implantable medical device is properly affixed to the body tissue.

In one implementation of the mentioned embodiments of the inventive apparatus, the at least one anchor may be hollow, and the at least one discharge channel is provided within the at least one anchor.

In an alternative implementation of the mentioned embodiments of the inventive apparatus, the at least one discharge channel may be provided within a hollow wire provided adjacent to the at least one anchor. In one embodiment of this implementation, the hollow wire may be made of a biodegradable material.

In one embodiment, the anchor control of the mentioned embodiments of the inventive apparatus may comprise a pump which is controllable to force the contrast agent fluid through the discharge channel and the outlet.

In one embodiment of the implantable medical device, the at least one anchor is hollow, and wherein the at least one discharge channel is provided within the at least one anchor.

In one embodiment of the implantable medical device, the at least one discharge channel is provided within a hollow wire provided adjacent to the at least one anchor. In one implementation of this embodiment, the hollow wire is made of a biodegradable material.

In this embodiment, the reservoir may be disposed within the implantable medical device. Additionally, or alternatively, the reservoir may be attached to the implantable medical device, either permanently or temporarily.

In one variant, the above mentioned implantable device further comprises a biodegradable cap provided on the distal portion of the anchor and covering the outlet of the discharge channel, the biodegradable cap configured to dissolve after a predetermined period of time to allow contrast agent fluid to discharge from the outlet.

The contrast agent fluid, which is used and provided within the reservoir, is visible in and around the body tissue using an imaging device. Further, an amount of contrast agent fluid released that is visible may provide an indication of whether or not the implantable device is properly affixed to the body tissue.

In one variant of the implantable medical device, the reservoir may comprise a compressible, fluid absorbent member attached to the implantable medical device, wherein compression of the member forces contrast agent fluid through the discharge channel and outlet. The compressible, fluid absorbent member of this variant may comprise a sponge.

A further aspect of the present disclosure comprises a method for controlling attachment of an implantable medical device to body tissue, the method comprising the following steps:
- anchoring an implantable medical device to body tissue at an implantation site, the implantable medical device comprising a housing, at least one anchor having a proximal portion affixed to the housing and a distal portion capable of being affixed to body tissue, and at least one discharge channel associated with the at least one anchor, the at least one discharge channel in fluid communication with a reservoir having a contrast agent fluid contained therein, and the at least one discharge channel having an outlet at the distal portion of the at least one anchor;
- actuating a flow of the contrast agent fluid from the reservoir and through the discharge channel and out the outlet, wherein the contrast agent fluid is discharged into and around the body fluid;
- viewing the implantation site using an imaging technique to identify at least one of a pattern or a flow of the contrast agent fluid; and
- determining whether the implantable medical device is properly affixed to the body tissue based on the at least one pattern or flow of the contrast agent fluid.

Further embodiments, features, aspects, objects, advantages, and possible applications of the present invention could be learned from the following description, in combination with the Figures, and the appended claims.

The above and other objects, aspects, features, advantages and possible applications of the present invention will be more apparent from the following more particular description thereof, presented in conjunction with the following drawings, in which:
- FIG. 1: is a perspective view of an exemplary apparatus positioned next to a heart (*i.e*., tissue body) to which the apparatus may be attached, with the anchor separated from the implantable medical device.
- FIG. 2: is a side view of an exemplary apparatus in accordance with the present invention.
- FIG. 3A: shows an exemplary apparatus in accordance with the present invention that illustrates an implantable medical device properly seated/attached to body tissue.
- FIG. 3B: shows an exemplary apparatus that illustrates an implantable medical device improperly seated/attached to body tissue and perforating the body tissue.
- FIG. 4A: shows a cross-section view taken along line A-A in FIG. 2 illustrating an exemplary anchor having a lumen formed within a central portion of the anchor, which may be used as a discharge channel for a contrast agent routed therein.
- FIG. 4B: shows cross-sectional view taken along line A-A in FIG. 2 illustrating an exemplary solid anchor and an exemplary discharge channel being formed in a wire routed alongside an exemplary anchor.
- FIG. 5: shows an exemplary apparatus in the form of a fluid connector which connects the outlets with an implantation tool like an implantation catheter.

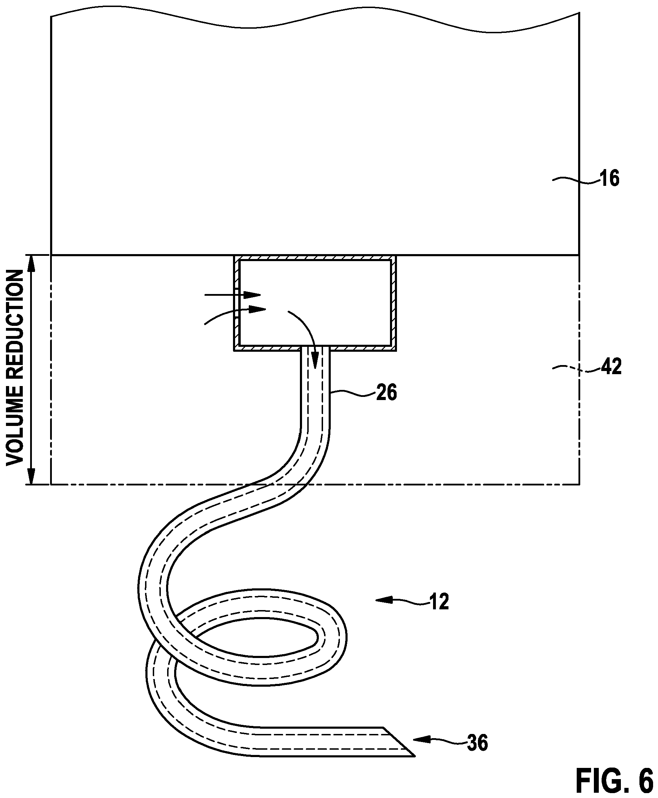

- FIG. 6: shows an exemplary apparatus with a cross-sectional view of an exemplary absorbent member as part of the apparatus.
- FIG. 7: is a side view of another exemplary apparatus in accordance with the present invention.
- FIG. 8: shows a cross-section view taken along line B-B in FIG. 7 illustrating an exemplary anchor with rectangular cross-section having a rectangular lumen formed within a central portion of the anchor, which may be used as a discharge channel for a contrast agent routed therein.

The following description of embodiments presently contemplated for carrying out the present invention. This description is not to be taken in a limiting sense, but is made merely for the purpose of describing the general principles and features of the present invention. The scope of the present invention should be determined with reference to the claims.

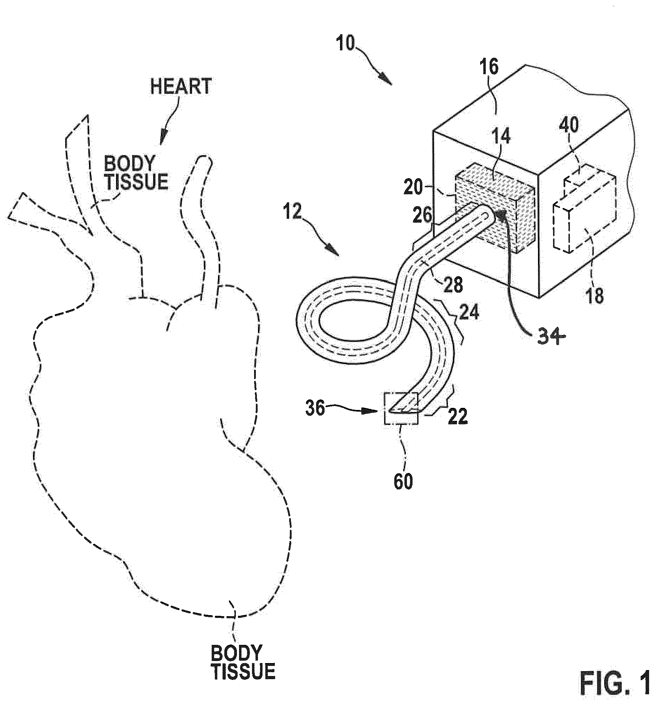

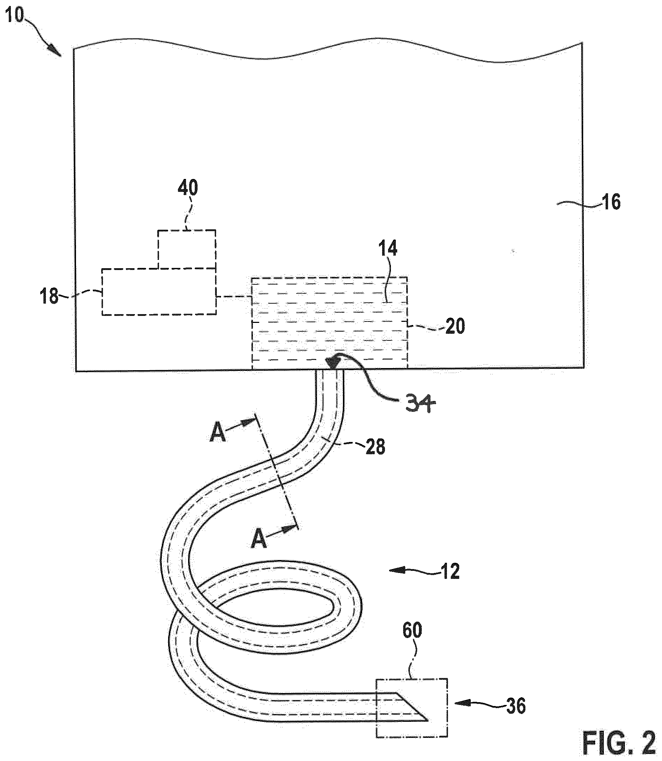

Referring to FIGS. 1-4, the apparatus 10 can include an anchor 12 configured to temporarily or permanently affix to body tissue and discharge a fluid 14 into and/or around the tissue. The anchor 12 may be configured to attach to various types of tissue, which can be any one or combination of epithelium tissue, muscle tissue, connective tissue, and/or nervous tissue, which may include cardiac tissue. In some embodiments, the anchor 12 can be further configured to temporarily or permanently affix to an implantable medical device 16. In at least one embodiment, the anchor 12 can be flexibly attached to the implantable medical device 16.

The implantable medical device 16 may include a casing/housing having components 18 housed therein and/or components 18 attached thereto. The components 18 of the implantable medical device may include electrical circuitry, pumps, transducers, transceivers, sensors, motors, actuators, etc. to diagnose, prevent, and/or treat physiological and other conditions of the living being and also to force the fluid 14 through the anchor, as described herein. The implantable medical device 16 may achieve this by monitoring and/or regulating organs or other components placed within or onto the being. For example, the implantable medical device 16 may be an implantable leadless pacemaker used to monitor and regulate the heart. The implantable medical device can also include other injectable devices, such as a venous access port or a neurostimulator (Vagus Nerve Stimulation, Spinal Cord Stimulation, Deep Brain Stimulation, for example), which also may be injectable, for example.

The fluid 14 (which may be a liquid, a solvable powder or a solvable solid object, or the like) is provided in a reservoir 20. As shown in FIGS. 1-2, the reservoir 20 may be provided within the implantable medical device 16. However, the reservoir 20 may also be attached to the implantable medical device 16 or otherwise provided outside of the device 16 without departing from the scope of the present invention. The only requirement is that the reservoir be in fluid communication with a discharge channel 28.

The implantable medical device 16 may be attached to the anchor 12 at a proximal portion of the anchor 12, while a distal portion of the anchor 12 can be used to attach the apparatus 10, along with the implantable medical device 16, to the body tissue. The anchor 12 is preferably contrast fluid enhanced (*i.e*., provided with a means to dispense contrast fluid) so as to dispense contrast fluid 14 while the apparatus 10 and the implantable medical device 16 are attached to the body tissue. The dispensing of contrast fluid, or agent, 14 may be controlled via a controller included within the components 18, such that the contrast fluid 14 can be dispensed automatically or on demand by a user.

The anchor 12 can include a first end 22 (distal end or distal portion), a shank 24, and a second end 26 (proximal end or proximal portion). The shank 24 is generally the body of the anchor that lies between the first end 22 and the second end 26. The anchor 12 may be a semi-rigid or rigid member fabricated from metal, plastic, carbon composite, fiberglass, ceramic, etc. It is contemplated for the anchor 12 to be configured to pierce body tissue and be retained therein, thus securing the medical device 16 thereto in a proper seating arrangement. This can be achieved by providing the anchor 12 with various shapes, lengths, widths, and sizes. For instance, if the implantable medical device 16 is intended to rest against the body tissue, the anchor 12 may be configured to enable attachment so as to facilitate the implantable medical device 16 resting against the body tissue with little or no volume of space existing between an interface of the implantable medical device 16 and a surface of the body tissue. As another example, if the implantable medical device 16 is intended to be attached to the body tissue but have a volume of space between a surface of the implantable medical device 16 and the body tissue, then the anchor 12 may be configured to enable attachment of the implantable medical device 16 in such a way.

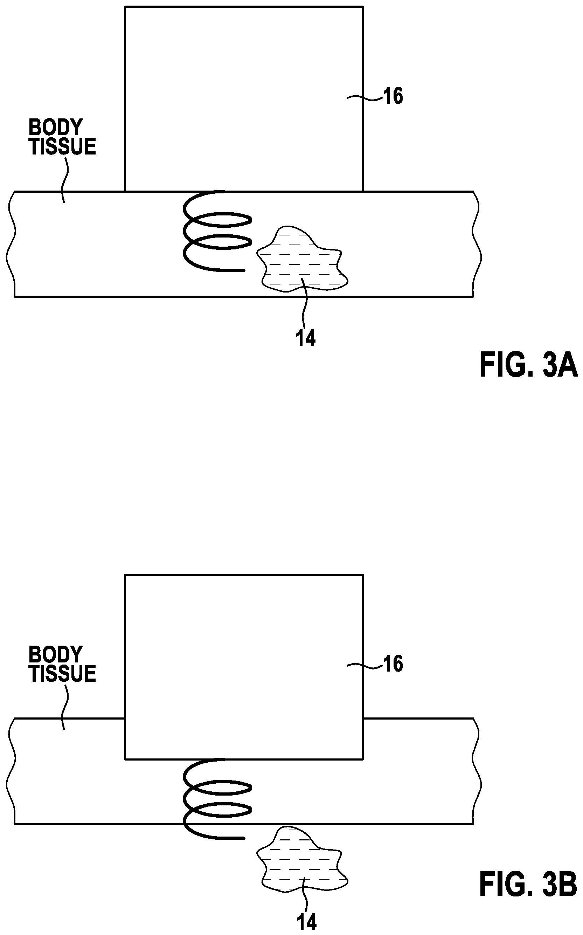

FIGS. 3A and 3B show an exemplary implantable medical device 16 being optimally implanted and improperly implanted where the anchor perforates the tissue-body, respectively. FIG. 3A shows proper attachment with the implantable medical device 16 anchored to the body tissue with the anchor 12 within the body tissue. FIG. 3B shows improper attachment with the implantable medical device 16 anchored to the body tissue with the anchor 12 perforating the body tissue. As will be explained in more detail hereafter, a discharge channel 28 is provided that is in or associated with the anchor 12 to facilitate dispensing of fluid 14 (contrast agent) therefrom. In FIG 3A, with the anchor 12 within the body tissue, the fluid 14 with diffuse into the body tissue. In FIG. 3B, with the anchor 12 perforating the body tissue, the fluid will diffuse into neighboring chambers or pericardial space. An imaging device (not shown) can be used to view the fluid 14 and determine if the implantable device 16 is properly attached or seated.

During implantation, the fluid 14 is kept in or at the medical device 16. After implantation, an active or passive process can start the release of fluid 14 from the device 16. For example, in FIG. 3A with the anchor 12 securely within the body tissue, the fluid 14 will be kept in the device 16 and/or elute slowly into the body tissue regions. In the case of a perforation, as shown in FIG. 3B, or another not well performed anchoring, the fluid 14 will elute relatively fast into close by cavities. Both of these conditions can be identified utilizing imaging apparatus, and the amount of fluid 14 visible can be used to determine if the device 16 is well anchored or not. The more fluid 14 that is visible, the more likely it is that the device is not well anchored and/or a perforation has occurred.

The first end 22 may be used to spearhead the incision of the anchor 12 into the body tissue, so the first end 22 may be structured as a pointed end to facilitate piercing tissue. The first end 22 may be referred to as the distal portion because in some embodiments the first end 22 is the portion of the apparatus 10 that is most distal to a practitioner when implanting an implantable medical device 16 with the apparatus 10.

The shank 24 may be a rigid or semi-rigid member structured to pierce tissue and affix itself thereto or therein. This may be by grappling the tissue, barbing the tissue, hooking the tissue, etc. In one embodiment, the shank 24 can include a coiled ridged member that is capable of being inserted into tissue and retained therein by a helicoid, helicoidal, or helical shape of the shank 24.

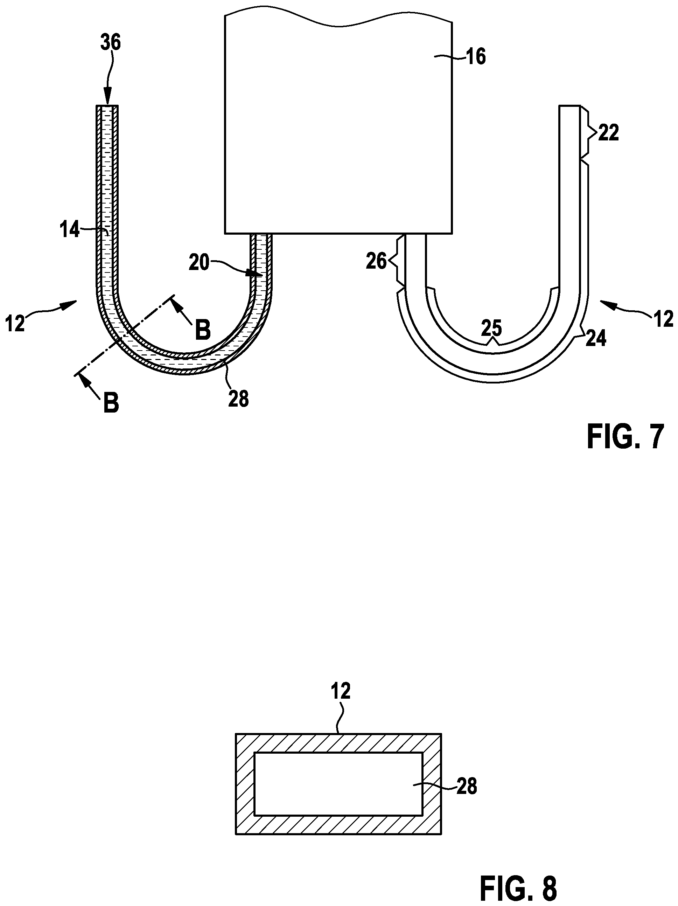

In another embodiment, the shank 24 is a semi-rigid member with a rectangular cross-section and can include a backward bend 25, which allows the anchor 12 to move backwards after incision of the tissue after release of a delivery device. An example is shown in FIG. 7, which discloses the distal end of an implantable medical device 16. At the distal end of the implantable medical device 16, at least two anchors 12 are fixedly attached. Each of the at least two anchors 12 comprise a first end 22, a shank 24 (which has a backward bent (portion) 25), and a second end 26, at which the at least two anchors 12 are fixedly attached to the medical device 16. Each of the at least two anchors 12 comprise a channel 28, which is filled with contrast agent fluid. In other words, the hollow space within the at least one anchor 12 comprises the reservoir 20 and is filed with one or more volatile substances (contrast agent fluid 14). These substances can be released directly through the outlet 36 and into the body tissue or cavity.

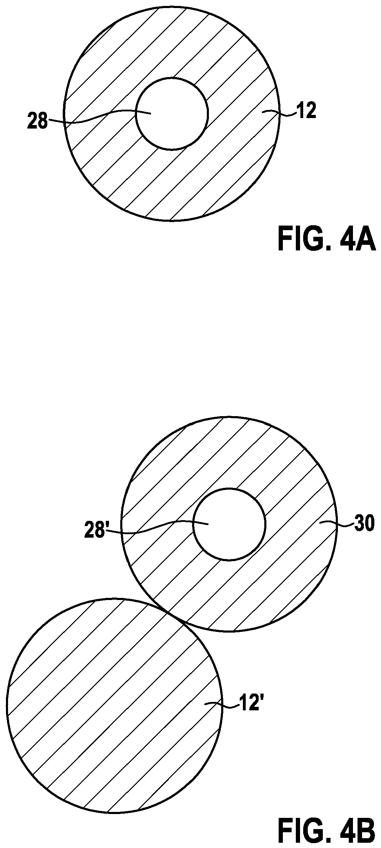

The shank 24 (and hence the anchor 12) may have a circular cross-section (*see* FIGS. 4A and 4B), but other cross-sections, like a flat wire with a rectangular cross-section (*see* FIG. 8), are contemplated. The shape, width, length, and pitch of the shank 24 may be selected based upon the desired application of the implantable medical device 16. In some embodiments, the shank 24 may be structured as a hook, a barb, a claw, a clamp, a screw, a straight member with at least one angled protrusion, etc. Once skilled in the art will appreciate, with the benefit of the present disclosure, that other of shapes, widths, lengths, and pitches may be utilized. For example, the anchor 12 may also take the form of a hook, clamp or claw.

The second end 26 may be structured as a securement end to temporarily or permanently attach to an ancillary object (*e.g.,* an implantable medical device 16). The second end 26 may be referred to as the proximal portion because in some embodiments the second end 26 is the portion of the apparatus 10 that is most proximal to a practitioner when implanting an implantable medical device 16.

In alternative embodiments, the anchor 12 can be integral to the implantable medical device 16. For example, the second end 26 can be molded from the casing/housing or other structure of the implantable medical device 16, and thus be integral to the implantable medical device 16.

The discharge channel 28 is provided either within or adjacent the anchor 12 and is configured to facilitate transfer of contrast agent fluid 14 there through. The discharge channel 28 may be formed within the anchor 12 (*i.e*., the anchor 12 is hollow), or formed in a separate member attached to the anchor 12. The discharge channel 28 has an outlet 36 at the distal end 22 of the anchor 12. As show in FIG. 4A, the anchor 12 is hollow and the discharge channel 28 is provided within the anchor 12. As shown in FIG. 4B, the anchor 12' is solid and the discharge channel 28' is provided within a wire or other member 30 attached to the anchor 12'. The outlet 36 of the discharge channel 28' in this embodiment is also at the distal end 22 of the anchor 12. However, depending on the particular application, the outlet 36 of the discharge channel 28, 28' may be at any portion of the anchor 12.

The discharge channel 28 is structured to be in fluid connection with at least one reservoir 20 (*see* FIGS.1-2). The reservoir 20 may be a container configured to retain the fluid 14 from which the discharge channel 28, 28' facilitates transfer of the fluid 14 therefrom. The reservoir 20 can be permanently or temporarily attached to a portion of the implantable medical device 16 or a portion of the anchor 12. The reservoir 20 can also be situated within the implantable medical device 16. For example, the reservoir 20 may be placed at the connector housing (header) or within the housing. Additionally, or alternately, to the outlets described above, further outlets can be provided in the header on the area which faces, in the implanted state, to the body tissue. Alternatively, the reservoir 20 can be separate from both the implantable medical device 16 and the anchor 12 and in fluid communication with the discharge channel 28, 28'. The reservoir 20 can also be configured to remain outside of the body while the implantable medical device 16 and/or anchor 12 is/are inserted into the body. It is contemplated for the reservoir 20 to be refillable.

The wire 30 can be structured as a tubular or flattened member, a hollow fiber, or other type of conduit with the discharge channel 28' running through it. The wire 30 can be fabricated from the same material as that of the anchor 12. Alternatively, the wire 30 can be fabricated from a biodegradable material so as to dissolve after an amount of time has elapsed. The biodegradation may be initiated once the biodegradable material comes into contact with the fluids of the being (*e.g*., blood). The biodegradable material may be, but is not limited to, Mg alloys, Zn alloys, Fe alloys, degradable polymers like Poly-L-Lactide acid and the like.

The wire 30 can be made to biodegrade after a certain amount of time has elapsed after the implantable medical device 16 has been implanted. For example, the apparatus 10 can be used during implantation and/or shortly thereafter to ascertain if the implantable medical device 16 has been properly seated/attached and/or if any tissue damage has occurred, but then the biodegradable wire 30 degrades after a period of time. The time frame can be chosen depending on the application of the implantable device and material used for the wire 30. This may be done to reduce possible risks involved with having a spent wire 30 within the tissue body. There can be an inherent risk, however slight, associated with leaving foreign objects in the body, thus it may be desirable to ensure that the spent wire 30 is dissolvable via biodegradation.

The discharge channel 28 may have an inlet end 34 attachable to the device 16 and in fluid communication with the reservoir 20. In some embodiments, a plurality of discharge channels 28, 28'. For example, the discharge channel 28 can be routed within the anchor 12, and the wire 30 with discharge channel 28' also provided adjacent the anchor 12. This can facilitate the dispensing of different types of fluid 14 by taking a different fluid 14 held in a different segmented portion of the reservoir 20. Alternately, the anchor 12 and/or wire 30 may have multiple discharge channels 28, 28' running there through.

In a preferred embodiment, the fluid 14 is a medical contrast medium and/or a contrast agent. A contrast agent can be any fluid 14 that enhances a contrast of bodily structures or bodily liquids during imaging. For example, a contrast agent may include a fluid 14 containing a substance that is radiopaque to enhance contrast between it and other structures and/or liquids when viewing the contrast agent and the other structures and/or liquids via X-ray imaging. Other contrast agents may generate a contrast effect with respect to MRI imaging, ultrasound imaging, or another imaging technique. A contrast agent may be a fluid 14 containing iodine, barium, gadolinium, etc. Contrast agents and/or other volatile substances can be dissolved in a liquid to form the fluid 14. The principles of the present invention are based on volatile substances like liquids or solvable powders, solvable solid objects and the like, which are visible with all known (and further developed) imaging methods, including X-ray, MRI imaging, etc. All of these substances are referred to herein as contrast agent.

In some embodiments, the apparatus 10 can include a pump 40 (*see* FIGS. 1-2) to force the fluid 14 through the discharge channel 28, 28' and out through the outlet end 36. The pump 40 can be located in the implantable device 16 (*see* FIGS. 1-2) or outside of the device. The pump 40 can be configured to draw the fluid 14 from, or push the fluid 14 out of, the reservoir 20 and force it through the discharge channel 28, 28'. The pump 40 may be also located within the reservoir 20.

Additionally, or alternatively, in some embodiments, the hollow space within the wire 30 or anchor 12 comprises the reservoir 20 and is filling with one or more volatile substances (fluid 14). These substances can be released directly through the outlet 36 and into the body tissue or cavity.

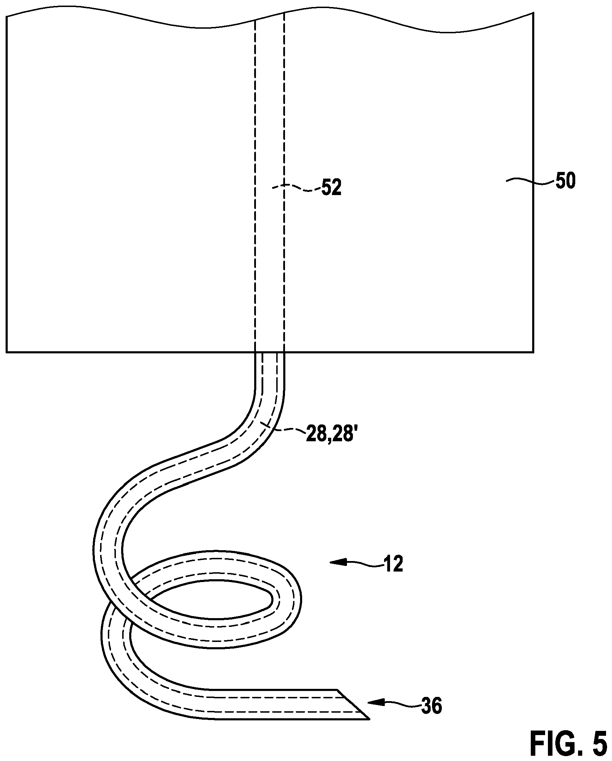

In one exemplary embodiment, the pump 40 is an internal pump, like a piezoelectric pump, placed at or near the reservoir 20. Of course, other pumps are contemplated. The pump 40 may be controlled by the surgeon either by wire through the implantation device or wirelessly so that the contrast agent (fluid 14) may be applied on demand. In some embodiments when a temporary fluid connector is used (*see* FIG. 5), the surgeon can control an external pump which pumps contrast agent fluid through the temporary fluid connector to the outlet.

A temporary fluid connector is shown in FIG. 5. The temporary fluid connector 50 is detachable and connects, via channel 52, the discharge channel 28, 28' with an implantation tool like an implantable catheter. The fluid 14 can be provided from an external reservoir and, in such case, the amount of fluid 14 is not limited.

In another embodiment, the outlet 36 can include a temporary cap 60 (*see* FIGS 1-2) that can be removed (for example, by dissolving) on demand and/or after a predetermined period of time (*e.g.,* 5-15 minutes). The temporary cap 60 may be fabricated from any of the biodegradable materials described above, such as Mg alloys, Zn alloys, Fe alloys, degradable polymers like Poly-L-Lactide acid and the like.

In a further exemplary embodiment, the reservoir 20 may be replaced with a compressible, fluid absorbent member 42 which is saturated with the fluid 14 (contrast agent) and acts as the reservoir. In one form, the compressible, fluid absorbent member 42 may be a sponge. As the member 42 is compressed and squeezed (for example, during implantation of the injectable device) a volume reduction of the member 42 occurs which forces fluid 14 into the proximal end 26 of the anchor 12 and out through the outlet 36. Upon release of the absorbent member 42, the absorbent member 42 can rebound back to an uncompressed state so that fluid 14 may be drawn back into the absorbent member 42 for subsequent dispensing of fluid 14.

An exemplary method of using the apparatus 10 may include use with an implantable medical device 16 (*e.g.,* an implantable leadless pacemaker). The second end 26 of the anchor 12 can be attached to a portion of the implantable medical device 16. An incision can be made into the living being, generating a pathway to the body tissue (*e.g.,* a heart) and creating an implantation site. The apparatus 10, along with the implantable medical device 16, can be introduced into the living being through the incision so that the first end 22 of the anchor 12 is distal to the user introducing the apparatus 10. The implantable medical device 16 can then be affixed to the body tissue by causing the first end 22 of the anchor 12 to pierce the tissue of the body tissue and manipulating (*e.g*., twisting) the anchor 12 to retain the implantable medical device 16 adjacent to the tissue-body, thus completing implantation. During implantation, the fluid 14 may be caused to remain in the reservoir 20. After or during implantation, active dispensing of fluid 14 can commence by a user (or an automated feature) activating the pump 40 or other device to force or pull fluid 14 through the discharge channel 28, 28' and out the outlet end 36. A user may then view the implantation site using an imaging technique to identify any patterns or flows of fluid 14. A user can determine that the implantable medical device 16 has been properly seated/attached by observing non-contrast effect due to no or little fluid 14 being visible and/or a slow contrast effect due to slow dispensing and elution of fluid 14 into tissue of the body tissue. A user can determine that the implantable medical device has not been properly seated/attached by observing a visually apparent contrast effect due to quick dispensing and elution of fluid 14 into a nearby body-cavity. If a perforation has occurred, the user can take immediate and appropriate action.

## Claims

1. A pacemaker system comprising an implantable leadless pacemaker comprising a housing, the pacemaker system further comprising an anchoring control system, wherein the anchoring control system comprises:
- at least one anchor (12, 12') having a proximal portion (26) affixed to the implantable leadless pacemaker (16) and a distal portion (22) capable of being affixed to body tissue;
- a reservoir (20) having a contrast agent fluid (14) contained therein; and
- at least one discharge channel (28, 28') associated with the at least one anchor, the at least one discharge channel being in fluid communication with the reservoir and having an outlet at the distal portion of the at least one anchor,
wherein contrast agent fluid is provided through the discharge channel and output at the outlet (36) to determine whether the implantable leadless pacemaker (16) is properly affixed to the body tissue.

2. The pacemaker system of claim 1, wherein the at least one anchor (12) is hollow, and wherein the at least one discharge channel (28) is provided within the at least one anchor.

3. The pacemaker system of claim 1 or 2, wherein the at least one discharge channel (28') is provided within a hollow wire (30) provided adjacent to the at least one anchor (12').

4. The pacemaker system of claim 3, wherein the hollow wire (30) is made of a biodegradable material.

5. The pacemaker system of claim 1, wherein the contrast agent fluid (14) is visible in and around the body tissue using an imaging device.

6. The pacemaker system of claim 1 or 5, wherein an amount of contrast agent fluid (14) released that is visible provides an indication of whether or not the implantable leadless pacemaker (16) is properly affixed to the body tissue.

7. The pacemaker system of claim 1, wherein the reservoir (20) is disposed within the implantable leadless pacemaker (16).

8. The pacemaker system of one of the preceding claims, further comprising a pump (40) which is controllable to force the contrast agent fluid (14) through the discharge channel (28, 28') and the outlet (36).

9. The pacemaker system of claim 1, wherein the reservoir comprises a compressible, fluid absorbent member (42) attached to the implantable leadless pacemaker (16), wherein compression of the member forces contrast agent fluid (14) through the discharge channel (28, 28') and outlet (36).

10. The pacemaker system of claim 9, wherein the compressible, fluid absorbent member (42) comprises a sponge.

11. The pacemaker system of one of the preceding claims, further comprising a biodegradable cap (60) provided on the distal portion (22) of the anchor (12) and covering the outlet (36) of the discharge channel (28, 28'), the biodegradable cap configured to dissolve after a predetermined period of time to allow contrast agent fluid (14) to discharge from the outlet.

## Patentansprüche

1. Schrittmachersystem, das einen implantierbaren anschlusslosen Schrittmacher aufweist, der ein Gehäuse aufweist, wobei das Schrittmachersystem ferner ein Verankerungssteuersystem aufweist, wobei das Verankerungssteuersystem Folgendes aufweist:
- wenigstens einen Anker (12, 12') mit einem proximalen Abschnitt (26), der am implantierbaren anschlusslosen Schrittmacher (16) befestigt ist, und einem distalen Abschnitt (22), der an Körpergewebe befestigt werden kann;
- einem Reservoir (20) mit einem darin enthaltenen Kontrastmittelfluid (14) und
- wenigstens einen Abflusskanal (28, 28'), der dem wenigstens einen Anker zugeordnet ist, wobei der wenigstens eine Abflusskanal mit dem Reservoir (20) in Fluidverbindung steht und einen Auslass am distalen Abschnitt des wenigstens einen Ankers hat,
wobei Kontrastmittelfluid durch den Abflusskanal bereitgestellt und am Auslass (36) ausgegeben wird, um zu bestimmen, ob der implantierbare anschlusslose Schrittmacher (16) ordnungsgemäß an Körpergewebe befestigt ist.

2. Schrittmachersystem nach Anspruch 1, wobei der wenigstens eine Anker (12) hohl ist und wobei der wenigstens eine Abflusskanal (28) in dem wenigstens einen Anker gebildet ist.

3. Schrittmachersystem nach Anspruch 1 oder 2, wobei der wenigstens eine Abflusskanal (28') in einem hohlen Kabel (30) bereitgestellt ist, das an den wenigstens einen Anker (12') angrenzend bereitgestellt ist.

4. Schrittmachersystem nach Anspruch 3, wobei das hohle Kabel (30) aus einem biologisch abbaubaren Material hergestellt ist.

5. Schrittmachersystem nach Anspruch 1, wobei das Kontrastmittelfluid (14) bei Verwendung einer bildgebenden Vorrichtung in und um das Körpergewebe sichtbar ist.

6. Schrittmachersystem nach Anspruch 1 oder 5, wobei eine freigesetzte Menge Kontrastmittelfluid (14), die sichtbar ist, einen Hinweis darauf gibt, ob der implantierbare anschlusslose Schrittmacher (16) ordnungsgemäß am Körpergewebe befestigt ist oder nicht.

7. Schrittmachersystem nach Anspruch 1, wobei das Reservoir (20) innerhalb des implantierbaren anschlusslosen Schrittmachers (16) angeordnet ist.

8. Schrittmachersystem nach einem der vorhergehenden Ansprüche, ferner eine Pumpe (40) aufweisend, die steuerbar ist, um das Kontrastmittelfluid (14) durch den Abflusskanal (28, 28') und den Auslass (36) abzugeben.

9. Schrittmachersystem nach Anspruch 1, wobei das Reservoir (20) ein zusammendrückbares fluidabsorbierendes Element (42) aufweist, das am implantierbaren anschlusslosen Schrittmacher (16) angebracht ist, wobei das Zusammendrücken des Elements Kontrastmittelfluid (14) durch den Abflusskanal (28, 28') und den Auslass (36) abgibt.

10. Schrittmachersystem nach Anspruch 9, wobei das zusammendrückbare fluidabsorbierende Element (42) einen Schwamm umfasst.

11. Schrittmachersystem nach einem der vorangehenden Asprüche, ferner eine biologisch abbaubare Kappe (60) aufweisend, die am distalen Abschnitt (22) des Ankers (12) bereitgestellt ist und den Auslass (36) des Abflusskanals (28, 28') bedeckt, wobei die biologisch abbaubare Kappe ausgelegt ist, sich nach einer vorbestimmten Zeitspanne aufzulösen, um Kontrastmittelfluid (14) aus dem Auslass abfließen zu lassen.

## Revendications

1. Système de stimulateur cardiaque comprenant un stimulateur cardiaque sans plomb implantable comprenant un boîtier, le système de stimulateur cardiaque comprenant en outre un système de commande d'ancrage, où le système de commande d'ancrage comprend :
- au moins un système d'ancrage (12, 12') ayant une partie proximale (26) fixée au niveau du stimulateur cardiaque sans plomb implantable (16) et une partie distale (22) capable d'être fixée au tissu corporel ;
- un réservoir (20) ayant un fluide d'agent de contraste (14) y étant contenu ; et
- au moins un canal de décharge (28, 28') associé avec l'au moins un système d'ancrage, l'au moins un canal de décharge étant en communication fluidique avec le réservoir et ayant une sortie au niveau de la partie distale de l'au moins un système d'ancrage,
où le fluide d'agent de contraste est délivré à travers le canal de décharge et est sorti au niveau de la sortie (36) afin de déterminer si le stimulateur cardiaque sans plomb implantable (16) est correctement fixé sur le tissu corporel.

2. Système de stimulateur cardiaque selon la revendication 1, dans lequel l'au moins un système d'ancrage (12) est creux, et dans lequel l'au moins un canal de décharge (28) est placé à l'intérieur de l'au moins un système d'ancrage.

3. Système de stimulateur cardiaque selon la revendication 1 ou 2, dans lequel l'au moins un canal de décharge (28') est placé à l'intérieur d'un câble creux (30) placé adjacent au niveau de l'au moins un système d'ancrage (12').

4. Système de stimulateur cardiaque selon la revendication 3, dans lequel le câble creux (30) est fabriqué dans une matière biodégradable.

5. Système de stimulateur cardiaque selon la revendication 1, dans lequel le fluide d'agent de contraste (14) est visible dans et autour du tissu corporel en utilisant un dispositif d'imagerie.

6. Système de stimulateur cardiaque selon la revendication 1 ou 5, dans lequel une quantité de fluide d'agent de contraste (14) libérée qui est visible procure une indication si oui ou non le stimulateur cardiaque sans plomb implantable (16) est correctement fixé au niveau du tissu corporel.

7. Système de stimulateur cardiaque selon la revendication 1, dans lequel le réservoir (20) est disposé à l'intérieur du stimulateur cardiaque sans plomb implantable (16).

8. Système de stimulateur cardiaque selon l'une des revendications précédentes, comprenant en outre une pompe (40) qui peut être commandée pour obliger le fluide d'agent de contraste (14) à traverser le canal de décharge (28, 28') et la sortie (36).

9. Système de stimulateur cardiaque selon la revendication 1, dans lequel le réservoir comprend un organe (42) compressible, adsorbant le fluide, rattaché au stimulateur cardiaque sans plomb implantable (16), où la compression de l'organe oblige le fluide d'agent de contraste (14) à traverser le canal de décharge (28, 28') et la sortie (36).

10. Système de stimulateur cardiaque selon la revendication 9, dans lequel l'organe (42) compressible adsorbant de fluide comprend une éponge.

11. Système de stimulateur cardiaque selon l'une des revendications précédentes, comprenant en outre un capuchon biodégradable (60) mis en place sur la partie distale (22) du système d'ancrage (12) et couvrant la sortie (36) du canal de décharge (28, 28'), le capuchon biodégradable étant configuré pour se dissoudre après un intervalle de temps prédéterminé afin de permettre au fluide d'agent de contraste (14) de se déverser à partir de la sortie.
